# EUROPEAN PATENT APPLICATION

(11) **EP 4 332 211 A1**
(43) Date of publication of application: **06.03.2024**
(21) Application number: 21939392.3
(22) Date of filing: 23.12.2021
(51) Int. Cl.: C12M 3/00, C12M 1/12, C12N 5/07

(54) **CULTURE VESSEL AND CULTURE VESSEL-USING METHOD**

(30) Priority: 30.04.2021 JP 2021077868
(71) Applicant: Ushio Denki Kabushiki Kaisha, Tokyo 100-8150 (JP)
(72) Inventor: YAMANAKA, Makoto, Tokyo 100-8150 (JP)
(74) Representative: Maiwald GmbH
(86) International application number: PCT/JP2021/047948
(87) International publication number: WO 2022/230237

(57) **Abstract**

Provided are a culture vessel that makes it possible to simulate a cellular response in a manner similar to in vivo and collect a liquid secretion from cells at a high concentration and a culture vessel-using method. A culture vessel 1 includes: a supply port 22 to supply a liquid containing cells; a main flow path 21 connected to the supply port 22; a culture space 3 to culture the supplied cells, at least part of the culture space 3 being located in the main flow path 21; a collection port 24 to collect a liquid secretion from the cells cultured in the culture space 3; a communication path 5 to allow the culture space 3 and the collection port 24 to communicate with each other; and a separation part 40 to separate the cells and the liquid secretion, the separation part 40 being located in the communication path 5 so as to be adjacent to the culture space 3.

## Description

### TECHNICAL FIELD

The present invention relates to a culture vessel and a culture vessel-using method.

### BACKGROUND ART

In the liver, drugs or other compounds are absorbed into and metabolized by hepatocytes. Hepatic metabolites are excreted into bile canaliculi, reach the gallbladder, and are discharged from the body as feces through the intestines.

As a conventional method for collecting a liquid secretion from hepatocytes, a cell culture method using a cell culture insert is known.

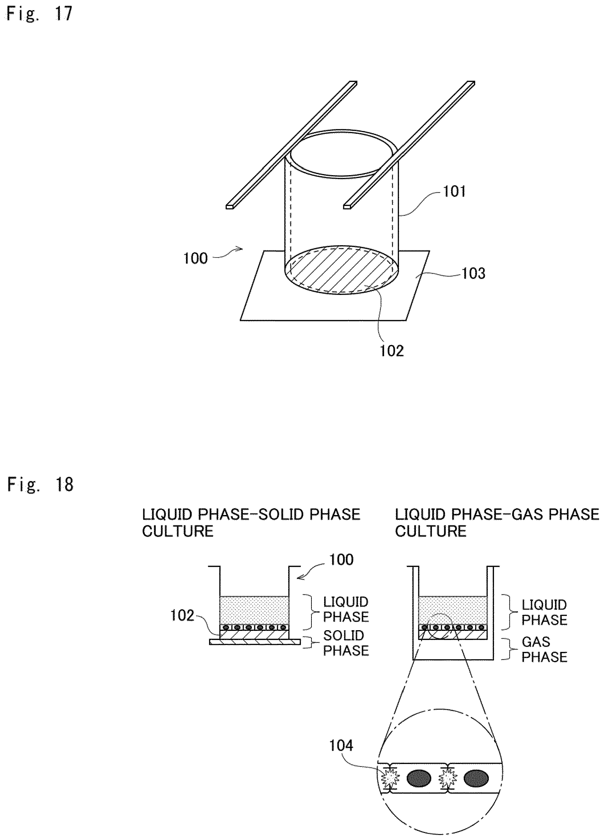

Fig. 17 shows an example of a conventional transportable culture vessel 100 having a membrane that can pass a physiologically active substance. A frame 101 has a space for holding cells inside a cylindrical tube made of acrylic or polystyrene, and a membrane 102 that can pass a physiologically active substance is fixed to one of the ends of the frame 101 by bonding with an adhesive. The membrane 102 has a releasable PET film 103 adhering thereto.

The membrane 102 that can pass a physiologically active substance is obtained by gelatinization of an artificial material made of a naturally occurring or synthetic polymer by introduction of cross-links. As an extracellular matrix (ECM) component to be gelatinized, for example, collagen, hyaluronic acid, gelatin, agar, agarose, or the like can be used.

As shown in Fig. 18, the culture vessel 100 having the membrane 102 that can pass a physiologically active substance can be transported while holding a culture such as cells therein to perform culture in different environments such as "liquid phase-membrane-solid phase", "liquid phase-membrane-gas phase", and "liquid phase-membrane-liquid phase".

Particularly, cell function assay can be performed using a transdermal absorption model or an intestinal absorption model having epithelial-mesenchymal interaction and obtained by respectively culturing epithelial cells and mesenchyme cells on one surface and the other surface of the membrane 102 that can pass a physiologically active substance, or an angiogenic model or a cancer invasion model obtained by respectively culturing vascular endothelial cells and cancer cells on one surface and the other surface of the membrane 102 that can pass a physiologically active substance.

Such transportability makes it possible to construct a hepatocyte culture device that promotes accumulation and excretion of hepatic metabolites in and into a bile canaliculus-like structure 104.

### PRIOR ART DOCUMENT

### PATENT DOCUMENT

Patent Document 1: JP-A-2020-28305

### SUMMARY OF THE INVENTION

### PROBLEMS TO BE SOLVED BY THE INVENTION

However, such a conventional culture vessel is large in volume, and therefore it is not possible to simulate a cellular response resulting from interaction between cells through a physiologically active substance in a manner similar to in vivo.

Further, a liquid secretion from cells is greatly diluted with a liquid in the culture device, and therefore it is difficult to collect a liquid secretion from cells at a high concentration.

In light of the above problems, it is an object of the present invention to provide a culture vessel that makes it possible to simulate a cellular response in a manner similar to in vivo and collect a liquid secretion from cells at a high concentration, and a culture vessel-using method.

### MEANS FOR SOLVING THE PROBLEMS

The present invention is directed to a culture vessel including:
a supply port to supply a liquid containing cells;
a main flow path connected to the supply port;
a culture space to culture the supplied cells, at least part of the culture space being located in the main flow path;
a collection port to collect a liquid secretion from the cells cultured in the culture space;
a communication path to allow the culture space and the collection port to communicate with each other; and
a separation part to separate the cells and the liquid secretion, the separation part being located in the communication path so as to be adjacent to the culture space.

Since this culture vessel includes the culture space in the main flow path, cells can be cultured in the culture space, and a liquid secretion from the cells can be separated from the cells by the separation part and collected, which makes it possible to simulate (reproduce, functionally reproduce, mimic, functionally mimic, model) a cellular response in a manner similar to in vivo and collect a liquid secretion from cells at a high concentration.

In the culture vessel according to the present invention, the supply port and the collection port may be formed to be opened in a first plane.

When the supply port and the collection port are formed to be opened in the same plane, supply of a liquid containing cells and collection of a liquid secretion can be performed on the same side so that excellent workability is achieved.

Further, in the culture vessel according to the present invention, the main flow path may be formed to extend in a second plane parallel to the first plane, and the communication path may be formed to be adjacent to the main flow path in a direction perpendicular to the second plane and to extend in a third plane that is parallel to the second plane and that is on an opposite side of the first plane across the second plane.

When the supply port and the collection port are disposed in the first plane, the main flow path is disposed in the second plane, and the communication path is disposed in the third plane, the culture vessel can be designed to be compact.

The present invention is also directed to a culture vessel-using method that is a method of using the culture vessel described above, the method including:
a first step of supplying a liquid containing cells through the supply port;
a second step of fixing the supplied cells in the culture space;
a third step of culturing the cells in the culture space; and
a fourth step of separating a liquid secretion from the cultured cells and the cells by the separation part; and
a fifth step of collecting the separated liquid secretion through the collection port.

The culture vessel-using method makes it possible to simulate (reproduce, functionally reproduce, mimic, functionally mimic, or model) a cellular response in a manner similar to in vivo and collect a liquid secretion from cells at a high concentration.

### BRIEF DESCRIPTION OF THE DRAWINGS

Fig. 1 is a plan view of a culture vessel according to an embodiment of the present invention.
Fig. 2Ais a sectional view of the culture vessel shown in Fig. 1 taken along line A-A.
Fig. 2B is a sectional view of the culture vessel shown in Fig. 1 taken along line B-B.
Fig. 2C is a sectional view of the culture vessel shown in Fig. 1 taken along line C-C.
Fig. 2D is a sectional view of the culture vessel shown in Fig. 1 taken along line D-D.
Fig. 3 is a perspective view around a third port.
Fig. 4 is an exploded perspective view of the culture vessel.
Fig. 5A is a schematic diagram for illustrating a culture vessel-using method.
Fig. 5B is a schematic diagram for illustrating the culture vessel-using method.
Fig. 5C is a schematic diagram for illustrating the culture vessel-using method.
Fig. 5D is a schematic diagram for illustrating the culture vessel-using method.
Fig. 6 is a sectional view of a culture vessel according to another embodiment.
Fig. 7 is a sectional view of a culture vessel according to another embodiment.
Fig. 8 is a sectional view of a culture vessel according to another embodiment.
Fig. 9A is a sectional view of a culture vessel according to another embodiment.
Fig. 9B is a perspective view of the culture vessel according to another embodiment.
Fig. 10 is a sectional view of a culture vessel according to another embodiment.
Fig. 11 is a sectional view of a culture vessel according to another embodiment.
Fig. 12 is a sectional view of a culture vessel according to another embodiment.
Fig. 13 is a sectional view of a culture vessel according to another embodiment.
Fig. 14 is a plan view of a culture vessel according to another embodiment.
Fig. 15A is a plan view of a culture vessel according to another embodiment.
Fig. 15B is a sectional view of the culture vessel according to another embodiment.
Fig. 15C is a schematic diagram for illustrating a method of using the culture vessel according to another embodiment.
Fig. 16A is a plan view of a culture vessel according to another embodiment.
Fig. 16B is a sectional view of the culture vessel according to another embodiment.
Fig. 16C is a schematic diagram for illustrating a method of using the culture vessel according to another embodiment.
Fig. 17 is a perspective view showing an example of a conventional culture vessel.
Fig. 18 is a sectional view showing a usage example of the conventional culture vessel.

### MODE FOR CARRYING OUT THE INVENTION

A culture vessel according to the present invention will be described with reference to the drawings. It should be noted that the drawings disclosed herein merely show schematic illustrations. Namely, the dimensional ratios on the drawings do not necessarily reflect the actual dimensional ratios, and the dimensional ratios are not necessarily the same between the drawings.

### [Structure]

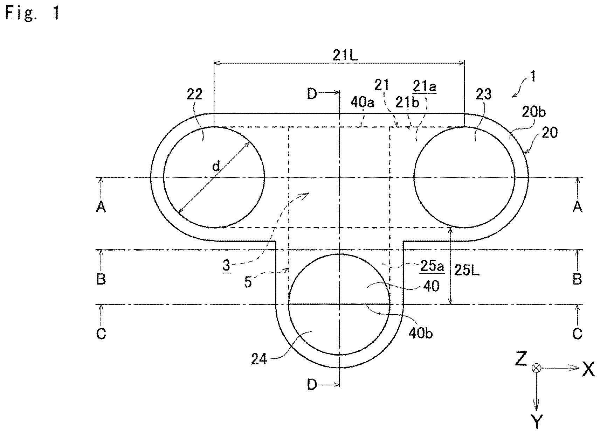

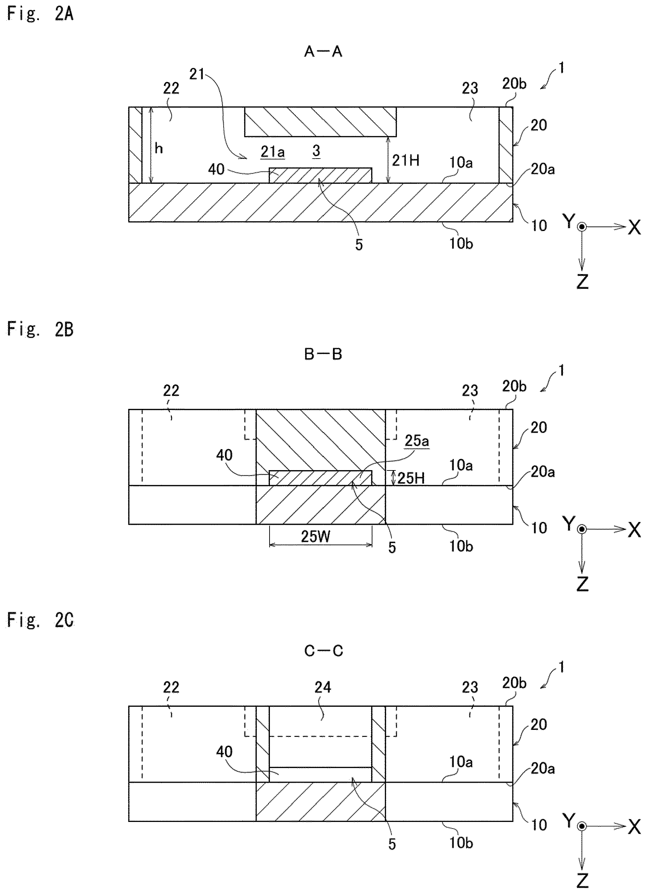

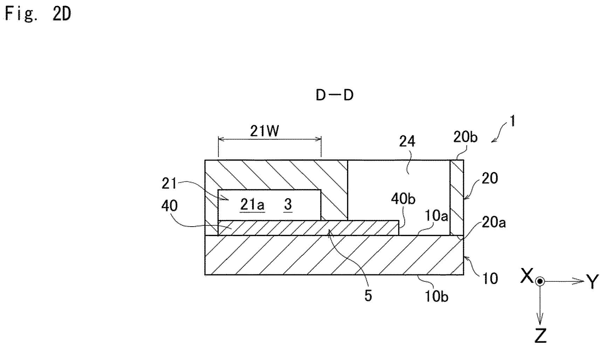

Fig. 1 is a plan view of a culture vessel 1 according to an embodiment of the present invention. Fig. 2A is a sectional view of the culture vessel 1 shown in Fig. 1 taken along line A-A. Fig. 2B is a sectional view of the culture vessel 1 shown in Fig. 1 taken along line B-B. Fig. 2C is a sectional view of the culture vessel 1 shown in Fig. 1 taken along line C-C. Fig. 2D is a sectional view of the culture vessel 1 shown in Fig. 1 taken along line D-D.

The culture vessel 1 is formed by stacking a second substrate 20 on a first substrate 10 so that one principal surface 20a of the second substrate 20 is partially in contact with one principal surface 10a of the first substrate 10 and bonding them together. The principal surface refers to one of surfaces constituting the substrate 10 or 20 and having a much larger area than other surfaces. The substrate 10 or 20 has two principal surfaces, and these two principal surfaces are opposed to each other.

In the following description, an XYZ coordinate system is appropriately referenced in which, in a state where the first substrate 10 and the second substrate 20 are bonded together, a plane parallel to the principal surfaces 10a and 10b of the first substrate 10 and the principal surfaces 20a and 20b of the second substrate 20 is defined as an XY plane and a direction orthogonal to the XY plane is defined as a Z direction.

When it is necessary to make a distinction between positive and negative to express a direction herein, the direction is described with a positive or negative sign, such as "+X direction" or "-X direction". When it is not necessary to make a distinction between positive and negative to express a direction, the direction is simply described as "X direction". Namely, in the present specification, in a case where a direction is simply described as "X direction", both "+X direction" and "-X direction" are included. The same applies to a Y direction and a Z direction. It should be noted that the culture vessel 1 is usually used so that the Z direction corresponds to a vertical direction, and the -Z direction corresponds to an upward direction.

The first substrate 10 and the second substrate 20 are the same in the shape of principal surfaces. In the culture vessel 1 of the present embodiment, the first substrate 10 and the second substrate 20 have an almost T-shape when viewed from the Z direction. The thickness of the second substrate 20 is larger than that of the first substrate 10. The thickness of the first substrate 10 is, for example, 0.1 to 1 mm, and the thickness of the second substrate 20 is, for example, 3 to 10 mm.

The principal surface 20a of the second substrate 20 has a first recess 21a. The principal surface 20a of the second substrate 20 has a second recess 25a. The other principal surface 20b of the second substrate 20 is located on the opposite side from the first substrate 10 and has first to third ports 22 to 24 (which will be described later).

The first recess 21a is formed in the principal surface 20a so as to extend in the X direction. When the first substrate 10 and the second substrate 20 are bonded together, the first recess 21a functions as a hollow main flow path 21 sandwiched between both of the substrates 10 and 20.

The first recess 21a has a slit shape that extends in the X direction so as to have constant width and depth. The first recess 21a has a rectangular sectional shape.

The first recess 21a has a width 21W (see Fig. 2D) of, for example, 2 to 10 mm in the Y direction. The first recess 21a has a depth 21H (see Fig. 2A) of, for example, 1 to 5 mm. The first recess 21a has a length 21L (see Fig. 1) of, for example, 8 to 30 mm.

The second recess 25a is formed in the principal surface 20a so as to extend in the Y direction. When the first substrate 10 and the second substrate 20 are bonded together, the second recess 25a functions as part of a hollow communication path 5 (which will be described later) sandwiched between both of the substrates 10 and 20.

The second recess 25a has a slit shape that extends in the Y direction so as to have constant width and depth. The second recess 25a is connected to the first recess 21a. The second recess 25a has a rectangular sectional shape.

The second recess 25a has a width 25W (see Fig. 2B) of, for example, 2 to 10 mm in the X direction. The second recess 25a has a depth 25H (see Fig. 2B) of, for example, 10 to 100 µm. The second recess 25a has a length 25L (see Fig. 1) of, for example, 1 to 10 mm.

The first port 22 is connected to the -X direction-side end of the first recess 21a, and is formed to extend from the principal surface 20a toward the principal surface 20b of the second substrate 20 and to penetrate the second substrate 20. The first port 22 is opened in the principal surface 20b. The second port 23 is connected to the +X direction-side end of the first recess 21a, and is formed to extend from the principal surface 20a toward the principal surface 20b of the second substrate 20 and to penetrate the second substrate 20. The second port 23 is opened in the principal surface 20b.

The third port 24 is connected to the +Y direction-side end of the second recess 25a, and is formed to extend from the principal surface 20a toward the principal surface 20b of the second substrate 20 and to penetrate the second substrate 20. The third port 24 is opened in the principal surface 20b.

The first port 22, the second port 23, and the third port 24 are all cylindrical hollows extending in the Z direction. In the culture vessel 1 of the present embodiment, the first port 22, the second port 23, and the third port 24 are all the same in diameter d (see Fig. 1), and the diameter d is, for example, 1 to 5 mm. It should be noted that it is not necessary for the first port 22, the second port 23, and the third port 24 to have the same diameter d. The first port 22, the second port 23, and the third port 24 are all the same in height h (see Fig. 2A), and the height h is, for example, 3 to 10 mm. The distance between the centerline of the main flow path 21 (first recess 21a) and the center of the third port 24 in the Y direction is, for example, 3 to 10 mm.

The first port 22 and the second port 23 are connected through the main flow path 21. That is, the main flow path 21 can be said to have the first port 22 and the second port 23 which are connected to the main flow path 21 and extend toward the principal surface 20b.

The first port 22 and the second port 23 have at least one of the purpose of supplying a liquid to the culture vessel 1 and the purpose of discharging a liquid from the culture vessel 1. For example, the first port 22 and the second port 23 may be used as a supply port and a discharge port, respectively.

The culture vessel 1 has a non-woven fabric 40 (which is an example of a separation part) in its inside. The non-woven fabric 40 is disposed across the first recess 21a and the second recess 25a. The non-woven fabric 40 has a rectangular plate shape that is long in the Y direction. One end 40a of the non-woven fabric 40 in the Y direction is located at a -Y direction-side edge 21b of the first recess 21a. Another end 40b of the non-woven fabric 40 in the Y direction is located at the center of the third port 24. The width of the non-woven fabric 40 in the X direction is the same as the width 25W of the second recess 25a in the X direction, and the thickness of the non-woven fabric 40 is the same as the depth 25h of the second recess 25a. That is, as shown in Fig. 2B, the sectional shape of the non-woven fabric 40 in an XZ plane is the same as that of the second recess 25a in the XZ plane.

The culture vessel 1 has a culture space 3 in its inside, and cells can be cultured in the culture space 3. The culture space 3 in the present embodiment is a space adjacent to the non-woven fabric 40 in the main flow path 21. However, cells may be cultured in a state where they slightly penetrate into the surface of the non-woven fabric 40. Although the details will be descried later, cells supplied to the main flow path 21 are mainly trapped on the bottom surface of the culture space 3, that is, on the upper surface of the non-woven fabric 40.

The culture vessel 1 has a communication path 5 to allow the culture space 3 and the third port 24 to communicate with each other. In the present embodiment, the space of the first recess 21a and the second recess 25a where the non-woven fabric 40 is disposed corresponds to the communication path 5.

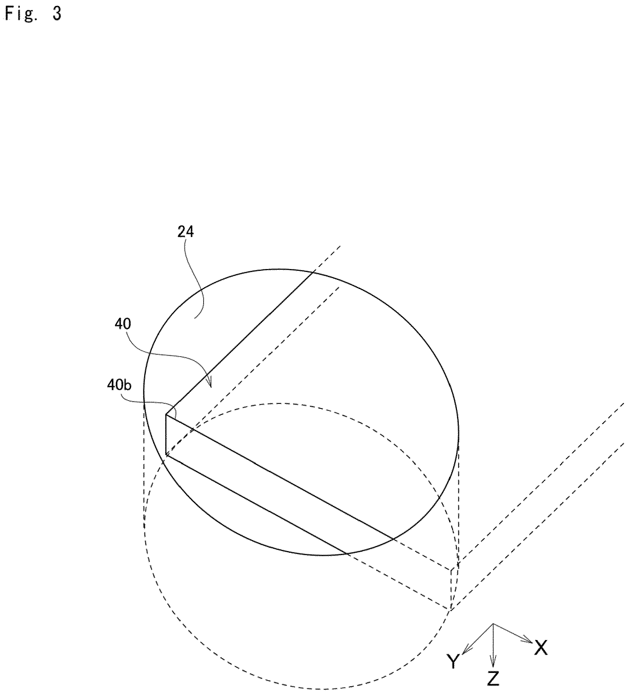

As shown in Fig. 3, the third port 24 is connected to the other end 40b of the non-woven fabric 40 which is the end of the communication path 5. That is, the communication path 5 can be said to have the third port 24 connected to the communication path 5 and extending toward the principal surface 20b.

The third port 24 has the purpose of discharging a liquid in the communication path 5 to the outside. For example, the third port 24 may be used as a collection port to collect a liquid in the communication path 5.

A liquid secretion from the cells cultured in the culture space 3 seeps out into the non-woven fabric 40 and then into the third port 24. At this time, most or all of the non-woven fabric 40 is covered with the cells, and therefore it is possible to almost completely or completely prevent a culture solution in the culture space 3 from flowing into the non-woven fabric 40. This makes it possible to collect the liquid secretion from the cells at a high concentration.

As described above, the culture vessel 1 according to the present embodiment includes the supply port (first port 22) to supply a liquid containing cells, the main flow path 21 connected to the supply port, and the culture space 3 to culture the supplied cells, at least part of the culture space 3 being located in the main flow path 21. Further, the culture vessel 1 includes the collection port (third port 24) to collect a liquid secretion from the cells cultured in the culture space 3 and the communication path 5 to allow the culture space 3 and the collection port to communicate with each other. In the communication path 5, the non-woven fabric 40 is disposed so as to be adjacent to the culture space 3. The non-woven fabric 40 functions as a separation part to separate cells cultured in the culture space 3 and a liquid secretion from the cells. Specifically, as the non-woven fabric 40, a material having a retained particle diameter smaller than the size of a general cell is used. More specifically, since a general cell has a diameter of about 10 to 50 µm, the retained particle diameter of the non-woven fabric 40 is preferably less than 20 µm, more preferably less than 10 µm. When a cluster of cells previously prepared is used, the diameter of the cluster of cells is about 50 to 300 µm, and therefore the retained particle diameter of the non-woven fabric 40 is preferably less than 100 µm, more preferably less than 50 µm.

In the culture vessel 1 according to the present embodiment, the surface of the non-woven fabric 40 may have cellular adhesiveness, and one of the surfaces of the non-woven fabric 40, which is in contact with the culture space 3, may have cellular adhesiveness. Cellular adhesiveness means the property of a surface having a chemical bond as a scaffold for cell adhesion or the property of a surface coatable with an ECM component (such as collagen, gelatin, or laminin), for example, a surface having a functional group such as a hydroxyl group or a carboxy group.

Such a configuration of the culture vessel 1 as described above makes it possible to simulate a cellular response in a manner similar to in vivo and collect a liquid secretion from cells at a high concentration, which will be described later with reference to a method of using the culture vessel 1.

### [Production method]

Hereinbelow, a method for producing the culture vessel 1 will be described in detail. It should be noted that in Fig. 1 to Fig. 2D, the first substrate 10 and the second substrate 20 have an almost T-shape when viewed from the Z direction. However, the first substrate 10 and the second substrate 20 are not limited thereto. In the following description of the production method, the first substrate 10 and the second substrate 20 have a rectangular shape when viewed from the Z direction.

### (Substrate preparing step)

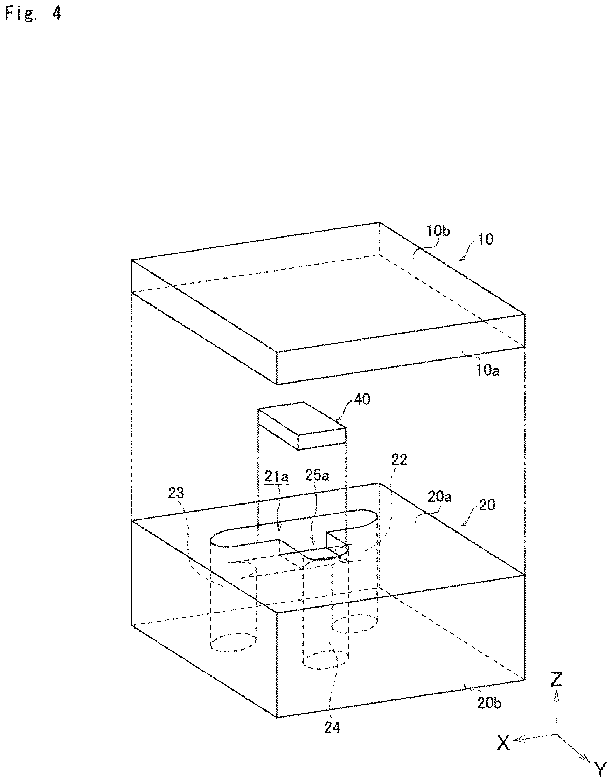

The first substrate 10, the second substrate 20, and the non-woven fabric 40 to form the culture vessel 1 are prepared. Fig. 4 is an exploded perspective view of the culture vessel 1.

A material used to form the substrates 10 and 20 is preferably a substantially non-porous material. The "substantially non-porous" herein refers to a state where the apparent surface area of the substrate is approximate to the actual surface area. Examples of a material to form such a non-porous material include an inorganic material such as glass or silicon and a resin material such as polymethyl methacrylate (PMMA), polycarbonate (PC), cycloolefin copolymer (COC), cycloolefin polymer (COP), polystyrene (PS), or silicone. It should be noted that these resin materials may be used in combination of two or more of them. The material used to form the first substrate 10 and the material used to form the second substrate 20 may be different.

The shape of the substrates in the present embodiment will be described. As the first substrate 10 and the second substrate 20, substrates that are the same in the shape of principal surfaces are used. The thickness of the second substrate 20 is larger than that of the first substrate 10. However, the first substrate 10 and the second substrate 20 may be different in the shape of principal surfaces. For example, the lengthwise dimension and widthwise dimension of principal surface of the first substrate 10 may be larger than those of principal surface of the second substrate 20, or the lengthwise dimension and widthwise dimension of principal surface of the second substrate 20 may be larger than those of principal surface of the first substrate 10. The thickness of the second substrate 20 may be the same as that of the first substrate 10, or the thickness of the second substrate 20 may be smaller than that of the first substrate 10.

The principal surface 20a of the second substrate 20 includes the first recess 21a and the second recess 25a. The other principal surface 20b of the second substrate 20 has openings as the first port 22, the second port 23, and the third port 24.

In order to provide openings and recesses in the second substrate 20, for example, a means such as injection molding or cutting work may be used, but an optimum means may be selected depending on the material forming the substrate. For example, as described above, when the second substrate 20 is formed using a resin material such as polymethyl methacrylate (PMMA), polycarbonate (PC), cycloolefin copolymer (COC), cycloolefin polymer (COP), polystyrene (PS), silicone, or acrylic, recesses can easily be formed by injection molding.

The non-woven fabric 40 is cut to have a predetermined size. The non-woven fabric 40 may be formed using silica or a polymer. Alternatively, the non-woven fabric 40 may be a commercially-available one such as Cell bed (trademark) manufactured by Japan Vilene Company, Ltd.

When the non-woven fabric 40 is thick, it takes time for a liquid secretion from cells (e.g., bile) to seep out. Therefore, the non-woven fabric 40 is preferably thin, and the thickness thereof is about 10 to 100 µm.

### (Substrate bonding step)

The principal surface 10a of the produced first substrate 10 and the principal surface 20a of the produced second substrate 20 are bonded together. The non-woven fabric 40 is disposed at a predetermined position when the first substrate 10 and the second substrate 20 are bonded together. A bonding method described below does not require formation of a thin film as an adhesive on the substrate and is performed in the following procedure.

First, the bonding surfaces (10a, 20a) of both of the substrates are subjected to surface activation treatment. As a method of the surface activation treatment, a method including irradiation with ultraviolet rays or a method including contact with plasma gas can be used.

The method including irradiation with ultraviolet rays is performed by, for example, irradiating the principal surface 20a of the second substrate 20 and the principal surface 10a of the first substrate 10 with vacuum ultraviolet rays having a wavelength of 200 nm or less emitted from an ultraviolet light source such as a xenon excimer lamp to emit light having a wavelength of 172 nm. As another example of the ultraviolet light source, a low-pressure mercury lamp having an emission line at 185 nm or a deuterium lamp having an emission line in a wavelength range of 120 to 200 nm can suitably be used. The irradiance of the vacuum ultraviolet rays is, for example, 10 to 500 mW/cm². An irradiation time is appropriately set depending on the type of resin used, and is, for example, 5 to 6 seconds.

The method including contact with plasma gas is performed by generating atmospheric-pressure plasma of a process gas containing nitrogen gas or argon gas as a main component and containing 0.01 to 5 vol% of oxygen gas and bringing the plasma into contact with the principal surface 20a of the second substrate 20 and the principal surface 10a of the first substrate 10. It is also possible to use a mixed gas of nitrogen gas and clean dry air (CDA). The time of contact with the plasma gas is, for example, 5 to 100 seconds.

Then, a bonding step is performed in which the first substrate 10 and the second substrate 20 are stacked so that the bonding surfaces (10a, 20a) of both of the substrates subjected to surface activation treatment are in contact with each other, and both of the substrates are bonded together by pressing using a press machine. The bonding step should be performed within a predetermined time, for example, within 10 minutes after the completion of an ultraviolet irradiation step in order to maintain the surface activation state.

If necessary, the bonding step is performed in a thermal environment to achieve tight bonding. In the bonding step, bonding conditions such as heating temperature and pressing force are set depending on the constituent material of the first substrate 10, the constituent material of the second substrate 20, and the constituent material of the non-woven fabric 40. As for specific conditions, the temperature during pressing is, for example, 40 to 130°C, and the pressing force for bonding is, for example, 0.1 to 10 MPa.

If necessary, a substrate obtained by bonding the first substrate 10 and the second substrate 20 together (hereinafter sometimes referred to as a "bonded substrate") may further be heated for a predetermined time after pressurization for a predetermined time. Even when a portion achieving a satisfactory bonding state and a portion not achieving a satisfactory bonding state are mixed at a bonded interface between the stacked substrates after pressurization, a desired bonding state can be achieved in the portion not achieving a satisfactory bonding state by heating the bonded substrate after pressurization.

After the pressurization of the bonded substrate is maintained for a predetermined time and then stopped, the temperature of the bonded substrate may be increased to and maintained at a predetermined temperature until a desired bonding state is achieved. Here, the predetermined temperature is a temperature at which deformation of the bonded substrate does not occur. For example, a heating temperature is 40 to 130°C and a heating time is 60 to 600 seconds.

Then, the culture vessel 1 in which the second substrate 20 is bonded onto the principal surface 10a of the first substrate 10 is produced through a cooling step.

### [Method of use]

Hereinbelow, a method of using the culture vessel 1 will be described in detail.

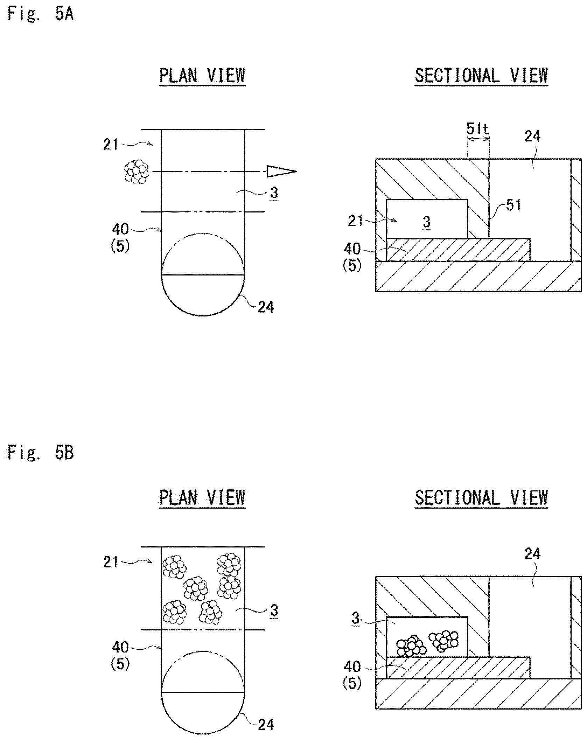

Figs. 5A to 5D are schematic diagrams for illustrating Usage Example 1 of the culture vessel 1, in which plan views are shown on the left side and sectional views are shown on the right side. It should be noted that the plan views on the left side are views of the inside of the culture vessel 1 when viewed from the Z direction.

i) First, as shown in Fig. 5A, hepatocytes are charged through the supply port (e.g., the first port 22) and inoculated in the main flow path 21 (first step). At this time, the hepatocytes are charged as clusters of cells. The size of each of the clusters of cells is, for example, about 50 to 300 µm.

As shown in Fig. 5B, the clusters of cells introduced into the main flow path 21 are trapped by the non-woven fabric 40 (second step). At this time, the cells are trapped so as to cover the upper surface of the non-woven fabric 40.

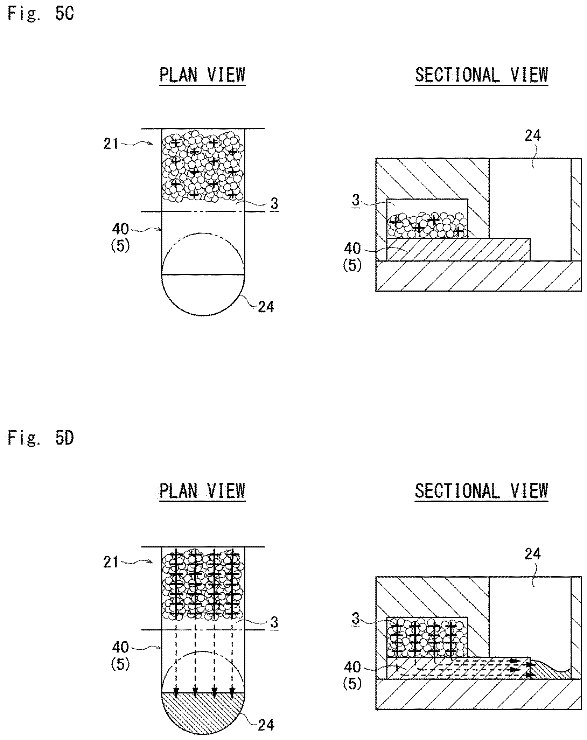

ii) Then, as shown in Fig. 5C, a culture solution is perfused by injecting it through the supply port and collecting it through the discharge port (e.g., the second port 23) to culture the hepatocytes (third step). The hepatocytes can be cultured in an environment similar to in vivo because the main flow path 21 has a geometrically small size.

The cells grow so that most or all of the upper surface of the non-woven fabric 40 is covered by the cells. As the hepatocytes grow, bile canaliculi (bile ducts) are formed in the hepatocytes.

iii) Then, a physiologically active substance or a drug of interest is charged through the supply port.

iv) Then, as shown in Fig. 5D, a liquid secretion (bile) from the bile canaliculi seeps out into the non-woven fabric 40 and then into the collection port (e.g., the third port 24) (fourth step). This makes it possible to separate the liquid secretion from the bile canaliculi and the cells. Then, the liquid secretion is collected through the collection port (fifth step). At this time, most or all of the non-woven fabric 40 is covered with the cells, and therefore it is possible to almost completely or completely prevent the culture solution in culture space 3 from flowing into the non-woven fabric 40. This makes it possible to collect the liquid secretion from the cells at a high concentration.

This method makes it possible to collect bile excreted from the bile canaliculi constructed between the hepatocytes. Further, it is possible to evaluate the metabolism of a physiologically active substance or a drug in hepatocytes in an environment simulating an in vivo environment.

In the culture vessel 1, the non-woven fabric 40 is preferably thin. Further, the contact surface between the non-woven fabric 40 and the culture space 3 is preferably large. Further, the communication path 5 is preferably short. In the present embodiment, a thickness 51t (see Fig. 5A) of a wall 51 between the culture space 3 and the collection port (third port 24) is preferably small. All of these are intended to reduce the time to allow bile to seep out.

As described above, the method of using the culture vessel 1 according to the present embodiment includes the first step of supplying a liquid containing cells through the supply port (first port 22), the second step of fixing the supplied cells in the culture space 3, the third step of culturing the cells in the culture space 3, the fourth step of separating a liquid secretion from the cultured cells and the cells by the non-woven fabric 40, and the fifth step of collecting the separated liquid secretion through the collection port (third port 24).

Although the embodiments of the present invention have been described above with reference to the drawings, it should be understood that specific configurations are not limited to these embodiments. The scope of the present invention is indicated not only by the above description of the embodiments but also by the claims, and all changes which come within the meaning and range of equivalency of the claims are therefore intended to be embraced therein.

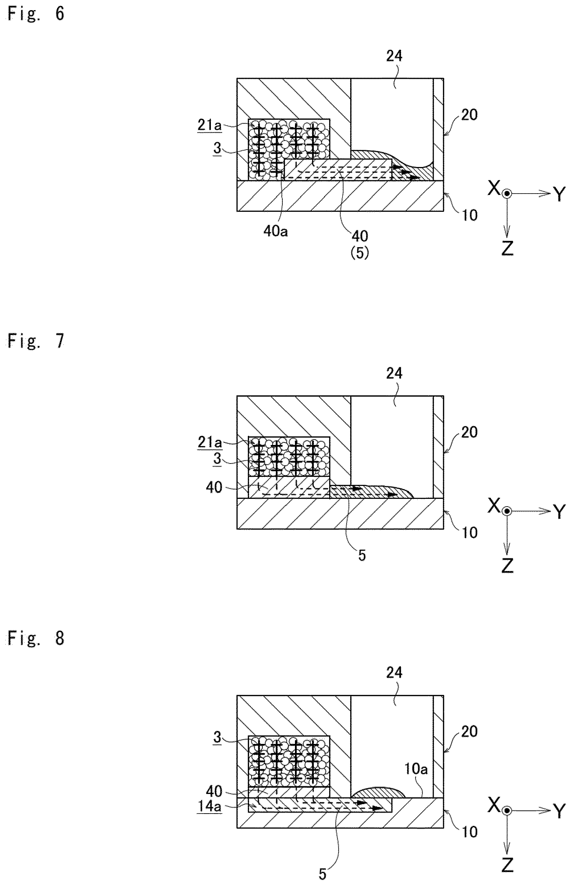

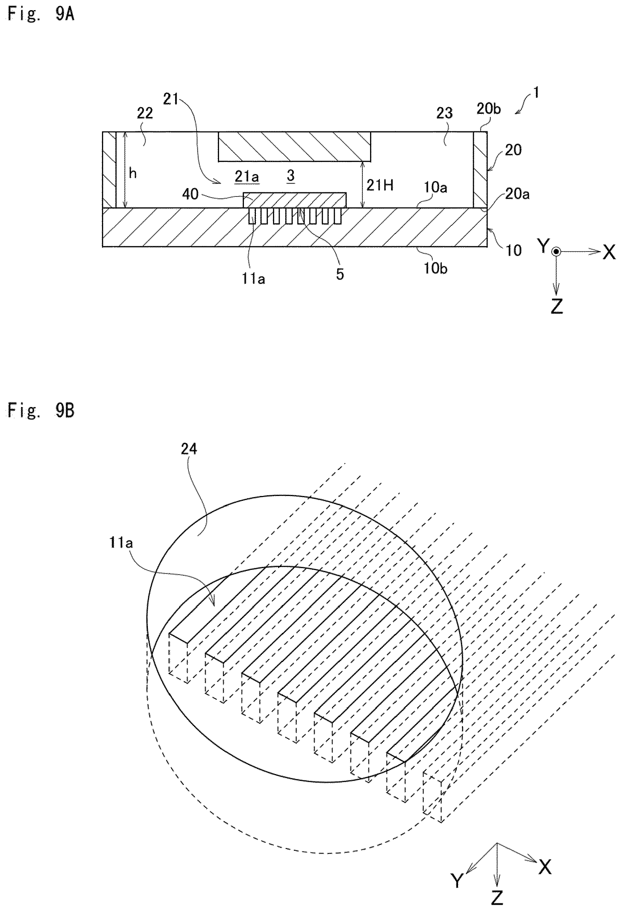

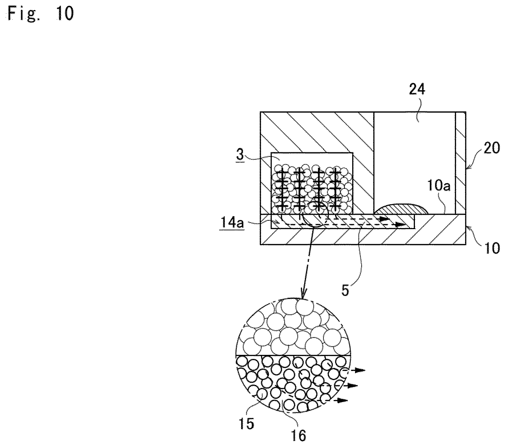

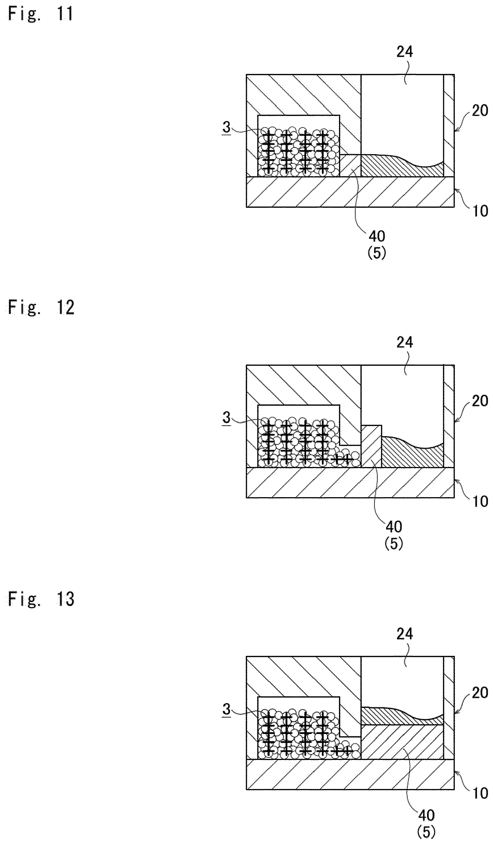

The structure adopted in each of the above embodiments can be adopted in any other embodiment. Specific configurations of parts are not limited only to those in the above-described embodiments, and various modifications can be made without departing from the spirit of the present invention.
(1) The above embodiment has been described with reference to a case where the one end 40a of the non-woven fabric 40 in the Y direction is located at the -Y direction-side edge 21b of the first recess 21a, but the present invention is not limited thereto. As shown in Fig. 6, the one end 40a of the non-woven fabric 40 may be located at the center of the first recess 21a in the Y direction.
(2) The above embodiment has been described with reference to a case where the non-woven fabric 40 is disposed throughout the communication path 5 that allows the culture space 3 and the collection port (third port 24) to communicate with each other, but the present invention is not limited thereto. As shown in Fig. 7, the non-woven fabric 40 may be disposed only at part of the communication path 5. In this case, the entirety of the non-woven fabric 40 is disposed in the first recess 21a.
(3) The above embodiment has been described with reference to a case where the second recess 25a formed in the principal surface 20a of the second substrate 20 functions as part of the communication path 5, but the present invention is not limited thereto. As shown in Fig. 8, a third recess 14a formed in the principal surface 10a of the first substrate 10 to extend in the Y direction may function as part of the communication path 5. At this time, the communication path 5 is adjacent to the main flow path 21 in the Z direction and extends in a different XY plane different from the main flow path 21. The +Y direction-side end of the third recess 14a is allowed to communicate with the third port 24. The width of the third recess 14a in the X direction is almost the same as the width of the non-woven fabric 40 in the X direction, and the third recess 14a is filled with, for example, a gel to support the non-woven fabric 40 from below. The liquid secretion from the cells seeps out into the third port 24 through the non-woven fabric 40 and the gel in the third recess 14a.
(4) As shown in Fig. 9A that is a sectional view taken along the same line as Fig. 2A, a plurality of grooves 11a formed in the principal surface 10a of the first substrate 10 to extend in the Y direction may function as part of the communication path 5. It should be noted that, similarly to the case shown in Fig. 8, the entirety of the non-woven fabric 40 is disposed in the first recess 21a. Fig. 9B is a perspective view of the third port 24 and its vicinity. The one ends of the grooves 11a communicate with the third port 24. A liquid secretion from cells flows into the grooves 11a through the non-woven fabric 40 and seeps out into the third port 24 through the one ends of the grooves 11a.
(5) In a case shown in Fig. 10, the third recess 14a shown in Fig. 8 is filled with a gel 16 in which beads 15 made of glass or ceramic are dispersed. The gel 16 may be charged into the third recess 14a by injecting a mixture of the beads 15 and the gel 16 obtained by mixing them at a predetermined ratio through the third port 24.
   In the case shown in Fig. 10, the non-woven fabric 40 is not provided, but the mixture of the gel 16 and the beads 15 functions as a separation part. The cells are supported by the mixture of the gel 16 and the beads 15, and a liquid secretion from the cells seeps out through the gel 16 between the beads 15 into the third port 24. The size of each of the beads 15 is desirably comparable to or smaller than that of a cell to be cultured.
(6) The non-woven fabric 40 may be disposed at, for example, any of positions shown in Fig. 11 to Fig. 13. In a case shown in Fig. 11, the non-woven fabric 40 is disposed in the second recess 25a. The communication path 5 is a space where the non-woven fabric 40 is disposed.

In a case shown in Fig. 12, the non-woven fabric 40 is disposed in the lower part of the third port 24. Part of the culture space 3 is located in the main flow path 21, and the rest is disposed in the second recess 25a. The communication path 5 is a space where the non-woven fabric 40 is disposed.

In a case shown in Fig. 13, the non-woven fabric 40 is disposed in the lower part of the third port 24 and covers the principal surface 10a of the first substrate 10. The communication path 5 is a space where the non-woven fabric 40 is disposed.

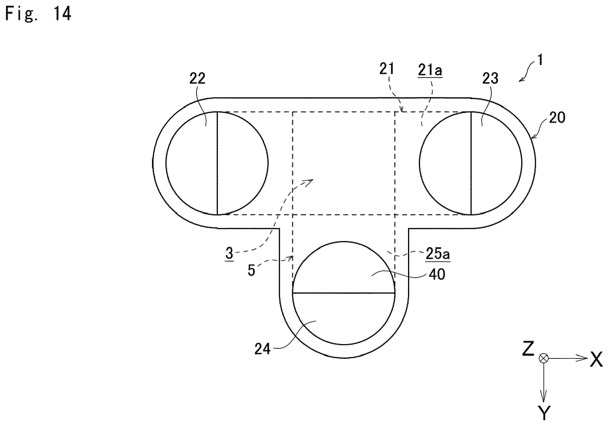

(7) As shown in Fig. 14, the non-woven fabric 40 may extend in the longitudinal direction of the first recess 21a (X direction). That is, the non-woven fabric 40 is disposed throughout the first recess 21a and the second recess 25a and has an almost T shape when viewed from the Z direction.

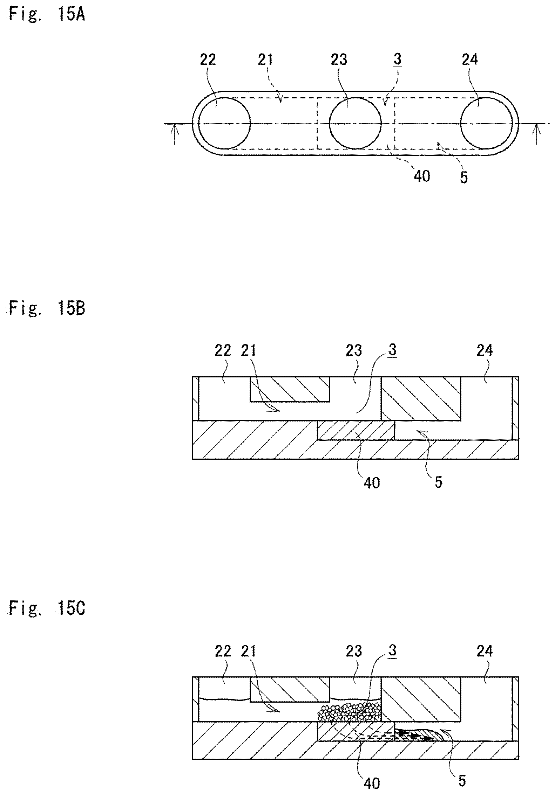

(8) Figs. 15A to 15C are respectively a plan view and a sectional view of a culture vessel according to another embodiment and a schematic view for illustrating a culture vessel-using method. In this case, the first port 22, the second port 23, and the third port 24 are arranged in a line. The main flow path 21, the non-woven fabric 40, and the communication path 5 extend in the same direction. The main flow path 21 and the communication path 5 are disposed at different positions in the vertical direction.

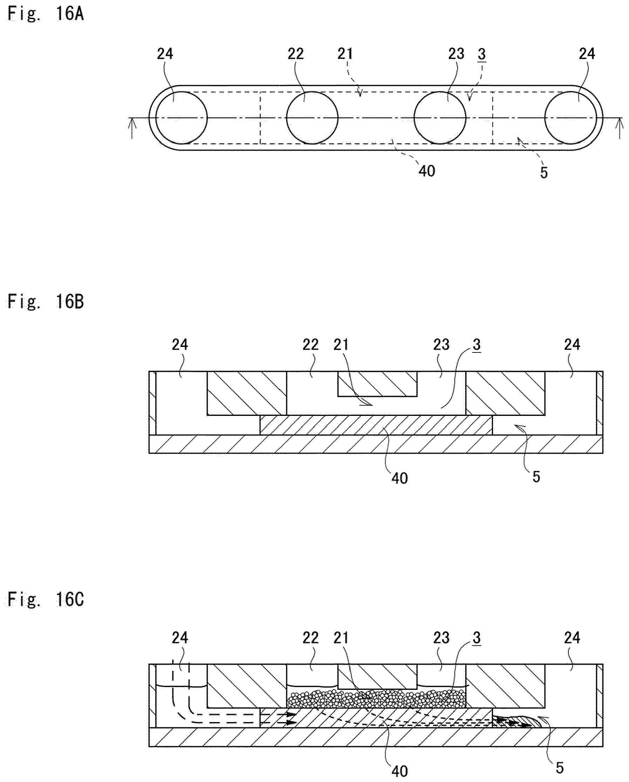

(9) Figs. 16A to 16C are respectively a plan view and a sectional view of a culture vessel according to another embodiment and a schematic view for illustrating a culture vessel-using method. In this case, the first port 22, the second port 23, and two third ports 24 are arranged in a line. The main flow path 21, the non-woven fabric 40, and the communication path 5 extend in the same direction. The main flow path 21 and the communication path 5 are disposed at different positions in the vertical direction. As shown in Fig. 16C, by injecting a liquid into one of the third ports 24, a liquid secretion (e.g., bile) seeping out into the non-woven fabric 40 is pushed out toward the other third port 24.

(10) In the above embodiment, hepatocytes (hepatic cells) are exemplified as cells to be cultured. However, parenchymal cells can be used as cells that form clusters. Interstitial cells can be used as cells that form membranes (layers).

Cells that form clusters are parenchymal cells, and examples thereof include hepatic parenchymal cells and pancreatic islet cells.

Cells that form membranes are interstitial cells, and examples thereof include epithelial and endothelial cells such as alveolar epithelial cells, tracheal/bronchial epithelial cells, gastrointestinal/intestinal epithelial cells, biliary epithelial cells, breast ductal epithelial cells, epidermal cells, mucosal epithelial cells, nasal cavity/pharynx epithelial cells, renal tubular epithelial cells, urothelial cells, corneal epithelial cells, retinal tissue cells, exocervical epithelial cells, fibroblast cells, and cancer cells thereof. Other examples include vascular endothelial cells and lymphatic endothelial cells.

When cells that form membranes are inoculated, clusters formed from the cells may be used.

Further, stem cells such as embryonic stem cells, adult stem cells, or iPS cells introduced into the culture area may be differentiated into such cells as described above. Such differentiated cells may also be used as cells to be cultured.

### DESCRIPTION OF REFERENCE SIGNS

- 1: Culture vessel
- 3: Culture space
- 5: Communication path
- 14a: Third recess
- 21: Main flow path
- 21a: First recess
- 22: First port
- 23: Second port
- 24: Third port
- 25a: Second recess
- 40: Non-woven fabric

## Claims

1. A culture vessel comprising:
a supply port to supply a liquid containing cells;
a main flow path connected to the supply port;
a culture space to culture the supplied cells, at least part of the culture space being located in the main flow path;
a collection port to collect a liquid secretion from the cells cultured in the culture space;
a communication path to allow the culture space and the collection port to communicate with each other; and
a separation part to separate the cells and the liquid secretion, the separation part being located in the communication path so as to be adjacent to the culture space.

2. The culture vessel according to claim 1, wherein the supply port and the collection port are opened in a first plane.

3. The culture vessel according to claim 2, wherein
the main flow path extends in a second plane parallel to the first plane, and
the communication path is adjacent to the main flow path in a direction perpendicular to the second plane and extends in a third plane that is parallel to the second plane and that is on an opposite side of the first plane across the second plane.

4. The culture vessel according to claim 1, wherein an entirety of the culture space is located in the main flow path, and part of the separation part is located in the main flow path.

5. The culture vessel according to claim 1, comprising a gel located in the communication path so as to be adjacent to the separation part.

6. The culture vessel according to claim 1, wherein the communication path has a plurality of grooves, each of which has both ends opened to the separation part and the collection port.

7. The culture vessel according to claim 1, wherein the separation part is constituted from a non-woven fabric.

8. The culture vessel according to claim 1, wherein the separation part is constituted from a gel in which beads are dispersed.

9. The culture vessel according to claim 3, wherein the communication path extends in a same direction as the main flow path.

10. The culture vessel according to claim 1, wherein a surface of the separation part which is in contact with the culture space has cellular adhesiveness.

11. A culture vessel-using method of using the culture vessel according to claim 1, the method comprising:
a first step of supplying a liquid containing cells through the supply port;
a second step of fixing the supplied cells in the culture space;
a third step of culturing the cells in the culture space; and
a fourth step of separating a liquid secretion from the cultured cells and the cells by the separation part; and
a fifth step of collecting the separated liquid secretion through the collection port.
